# EUROPEAN PATENT APPLICATION

(11) **EP 4 772 526 A1**
(43) Date of publication of application: **08.07.2026**
(21) Application number: 24880041.9
(22) Date of filing: 11.10.2024
(51) Int. Cl.: C07K 7/06, A61P 17/00, A61K 8/64, A61Q 19/00, A23L 33/18

(54) **NOVEL PEPTIDE HAVING ACTIVITY OF IMPROVING SKIN CONDITION AND USES THEREOF**

(30) Priority: 16.10.2023 KR 20230137618
(71) Applicant: Caregen Co., Ltd., Seoul 06188 (KR)
(72) Inventor: CHUNG, Yong Ji, Seoul 06188 (KR); KIM, Eun Mi, Seoul 06188 (KR); LEE, Eung Ji, Seoul 06188 (KR); KANG, Hana, Seoul 06188 (KR); HWANG, Bobyeol, Seoul 06188 (KR)
(74) Representative: Bandpay & Greuter
(86) International application number: PCT/KR2024/015420
(87) International publication number: WO 2025/084704

(57) **Abstract**

The present invention relates to a peptide exhibiting skin condition improvement activity. The peptide of the present invention increases the expression levels of extracellular matrix components and skin barrier enhancement factors, reduces the expression of skin cell damage factors increased by ultraviolet irradiation, and restores the expression levels of extracellular matrix components and skin barrier enhancement factors reduced by ultraviolet irradiation.

## Description

### TECHNICAL FIELD

### [CROSS-REFERENCE TO RELATED APPLICATIONS]

This application claims priority from Korean Patent Application No. 10-2023-0137618, filed on October 16, 2023, the disclosure of which is incorporated herein by reference in its entirety.

### [TECHNICAL FIELD]

The present invention relates to a novel peptide having a skin condition-improving activity and uses thereof.

### BACKGROUND ART

The skin is an organ that covers all organs and organ systems of the human body, protects the body from external invasion, and plays an important role in regulating homeostasis in the body by preserving moisture and temperature in the body. Histologically, the skin is composed of three layers: the epidermis, the dermis, and the subcutis. Aging of the skin may be divided into aging due to intrinsic factors and aging due to extrinsic factors. Aging due to intrinsic factors occurs naturally with age, and it has been reported that, for example, a decrease in activity of skin cells due to genetic causes such as telomere shortening is a cause thereof. Aging due to extrinsic factors occurs due to environmental factors such as sunlight, cold, wind, and fine dust, and it is known that, among these, ultraviolet rays and infrared rays of sunlight are the most important causes.

As aging of the skin progresses, collagen, which is a fibrous tissue serving to support the structure of the skin, and elastic fibers are hardened. Meanwhile, a ground substance that fills between cells and between fibrous materials such as collagen and elastic fibers has a remarkably high ability to retain moisture, and as such ground substances and the like decrease, elasticity of the skin decreases and wrinkles occur. Representative substances among such ground substances are hyaluronic acid and mucopolysaccharides, and as aging of the skin progresses, hyaluronidase increases, such that the amount of hyaluronic acid in the dermis gradually decreases. In addition, reactive oxygen species (free radicals) induced by ultraviolet rays or generated by intracellular respiration react with unsaturated fatty acids of cell membranes to form a new radical, a peroxyradical, and this substance causes a chain reaction in the presence of oxygen, thereby inducing lipid peroxidation again. Lipid peroxides may further amplify lipid peroxidation reactions and, by reacting with proteins, impair functions of enzyme or receptor systems. As described above, reactive oxygen species is a major cause of damage to cells and tissues of the skin. In addition, reactive oxygen species destroys the skin antioxidant defense system composed of antioxidant enzymes and non-enzymatic antioxidants, thereby shifting the balance between oxidants and antioxidants toward an oxidative state, and continued oxidative stress damages biological components through processes such as lipid peroxidation, protein oxidation, activation of proteolytic enzymes that destroy interstitial matrix components, chain cleavage and abnormal crosslinking of collagen and elastin, which are elastic fibers, cleavage of hyaluronic acid chains, promotion of melanogenesis, and DNA oxidation.

Patent documents relating to peptide active substances having activity of improving skin aging or skin wrinkles are as follows: Korean Patent No. 10-2486996, Korean Patent No. 10-2507392, and PCT International Patent Application No. PCT-KR2021-011278.

### [Related Art Documents]

### [Patent Documents]

Patent Document 1. Korean Patent No. 10-2486996
Patent Document 2. Korean Patent No. 10-2507392
Patent Document 3. PCT International Patent Application No. PCT-KR2021-011278

### DISCLOSURE OF THE INVENTION

### TECHNICAL PROBLEM

The present inventors have conducted studies to develop an effective active substance that may be used to inhibit skin aging and improve a skin condition such as improvement of skin wrinkles. As a result, the present inventors have experimentally confirmed that a novel peptide synthesized by them has an excellent skin condition-improving activity for preventing skin aging and skin wrinkles due to not only intrinsic factors but also extrinsic factors, thereby completing the present invention.

Accordingly, an object of the present invention is to provide a novel peptide having a skin condition-improving activity.

Another object of the present invention is to provide a composition for improving a skin condition, the composition containing the peptide as an active ingredient.

### TECHNICAL SOLUTION

In order to achieve the above-described object of the present invention,
one aspect of the present invention provides a peptide including the amino acid sequence set forth in SEQ ID NO: 1.

Another aspect of the present invention provides a composition for improving a skin condition, the composition containing the peptide as an active ingredient.

Hereinafter, the present invention will be described in detail.

### Peptide and Activity Thereof

According to one aspect of the present invention, there is provided a peptide including the amino acid sequence set forth in SEQ ID NO: 1.

### [Amino acid sequence of SEQ ID NO: 1]

### DRKKIQNK

As used herein, the term "peptide" means a linear molecule formed by amino acid residues linked to each other by peptide bonds.

The peptide including the amino acid sequence of SEQ ID NO: 1 of the present invention may be used without modification; however, an amino acid variant or fragment having a different sequence, obtained by deletion, insertion, substitution, or a combination thereof of amino acid residues, may be used within a range that does not affect the inherent activity of the peptide, for example, a skin condition-improving activity.

The peptide of the present invention may be modified by phosphorylation, sulfation, acrylation, glycosylation, methylation, farnesylation, and the like, within a range that does not change its activity.

The peptide of the present invention includes a peptide including the amino acid sequence of SEQ ID NO: 1, a peptide including an amino acid sequence substantially identical thereto, and variants thereof or active fragments thereof. The substantially identical amino acid sequence refers to an amino acid sequence having 75% or more, for example, 80% or more, 85% or more, 90% or more, 95% or more, 97% or more, or 98% or more sequence identity with the amino acid sequence of SEQ ID NO: 1. In addition, the peptide may further include a targeting sequence, a tag, a labeled residue, or an amino acid sequence prepared for a specific purpose of increasing a half-life or peptide stability.

The peptide of the present invention may be a peptide in which N-terminal and/or C-terminal modification is induced by selecting a partial region of an amino acid sequence in order to increase the activity thereof. Through such N-terminal and/or C-terminal modification, stability of the peptide of the present invention may be significantly improved, and, for example, a half-life of the peptide may be increased upon administration in vivo. The term "stability" includes not only in vivo stability for protecting the peptide of the present invention from attack by proteases in the body, but also storage stability (for example, stability during storage at room temperature).

The N-terminal modification may be one in which a protecting group selected from the group consisting of an acetyl group, a fluorenylmethoxycarbonyl group, a formyl group, a palmitoyl group, a myristyl group, a stearyl group, and polyethylene glycol (PEG) is attached to the N-terminus of the peptide. The C-terminal modification may be one in which a hydroxyl group (-OH), an amino group (-NH2), an azide (-NHNH2), or the like is attached to the C-terminus of the peptide, but is not limited thereto.

The peptide of the present invention may be prepared by various methods well known in the technical field to which the present invention pertains. For example, the peptide of the present invention may be prepared according to chemical synthesis methods known in the art, in particular, solid-phase synthesis techniques (Merrifield, J. Amer. Chem. Soc. 85:2149-54 (1963); Stewart et al., Solid Phase Peptide Synthesis, 2nd ed., Pierce Chem. Co., Rockford, 111 (1984)) or liquid-phase synthesis techniques (U.S. Patent No. 5,516,891).

The peptide of the present invention has activity of improving a skin condition.

In one embodiment, the peptide of the present invention has the following activities in relation to improving a skin condition:
(i) increasing expression levels of collagen Iα1, fibronectin, and elastin genes, which are extracellular matrix components;
(ii) increasing expression levels of SIRT1 (Sirtuin 1) and AQP3 (Aquaporin 3) genes, which are skin barrier enhancing factors;
(iii) increasing expression levels of collagen Iα1, fibronectin, and elastin genes, which are extracellular matrix components, the expression levels being reduced by UV irradiation;
(iv) increasing expression levels of SIRT1 and AQP3 genes, which are skin barrier enhancing factors, the expression levels being reduced by ultraviolet irradiation; and
(v) reducing expression levels of MMP-1 and MMP-2 increased by ultraviolet irradiation.

### Composition for Improving Skin Condition

According to another aspect of the present invention, there is provided a composition for improving a skin condition, the composition containing, as an active ingredient, a peptide including the amino acid sequence set forth in SEQ ID NO: 1.

The peptide of the present invention having the above-described activity exhibits excellent efficacy for improving a skin condition and thus may be used to improve a skin condition.

In one embodiment, in the composition for improving a skin condition, the improvement of the skin condition may be improvement in skin aging, promotion of skin regeneration, improvement in skin elasticity, improvement in skin wrinkles, or improvement in a skin barrier.

In one embodiment, the composition for improving a skin condition of the present invention has one or more of the following activities:
(i) increasing expression levels of collagen Iα1, fibronectin, and elastin genes, which are extracellular matrix components;
(ii) increasing expression levels of SIRT1 (Sirtuin 1) and AQP3 (Aquaporin 3) genes, which are skin barrier enhancing factors;
(iii) increasing expression levels of collagen Iα1, fibronectin, and elastin genes, which are extracellular matrix components, the expression levels being reduced by UV irradiation;
(iv) increasing expression levels of SIRT1 and AQP3 genes, which are skin barrier enhancing factors, the expression levels being reduced by ultraviolet irradiation; and
(v) reducing expression levels of MMP-1 and MMP-2 increased by ultraviolet irradiation.

The composition for improving a skin condition of the present invention may contain the peptide of the present invention at a concentration of 0.01 µM to 1,000 µM. Specifically, the peptide of the present invention may be contained at a concentration of 0.01 µM to 1,000 µM; 0.05 µM to 800 µM, 0.05 µM to 700 µM, 0.05 µM to 600 µM, 0.05 µM to 500 µM, 0.05 µM to 300 µM, or 0.05 µM to 200 µM; 0.1 µM to 800 µM, 0.1 µM to 700 µM, 0.1 µM to 600 µM, 0.1 µM to 500 µM, 0.1 µM to 300 µM, or 0.1 µM to 200 µM; 1 µM to 800 µM, 1 µM to 700 µM, 1 µM to 600 µM, 1 µM to 500 µM, 1 µM to 300 µM, or 1 µM to 200 µM; 5 µM to 800 µM, 5 µM to 700 µM, 5 µM to 600 µM, 5 µM to 500 µM, 5 µM to 300 µM, or 5 µM to 200 µM, but is not limited thereto.

In one embodiment, the composition for improving a skin condition described above may be a cosmetic composition.

The cosmetic composition may be prepared in any formulation commonly manufactured in the technical field to which the present invention pertains, and may be a topical preparation. For example, the cosmetic composition may be formulated in the form of a solution, a suspension, an emulsion, a paste, a gel, a cream, a lotion, a powder, a soap, a surfactant-containing cleanser, an oil, a powder foundation, an emulsion foundation, a wax foundation, or a spray, but is not limited thereto.

The cosmetic composition may be prepared in various forms such as a solution, a sol-gel, an emulsion, an oil, a wax, and an aerosol, including, but not limited to, a softening toner, a nourishing toner, a nourishing cream, a massage cream, an essence, an eye cream, a cleansing cream, a cleansing foam, a cleansing water, a pack, a spray, a powder, a hair tonic, a hair cream, a hair lotion, a hair shampoo, a hair rinse, a hair conditioner, a hair spray, a hair aerosol, a pomade, and a gel.

The cosmetic composition of the present invention may contain other additives, such as excipients and carriers, and may be formulated by appropriately incorporating, as needed, conventional ingredients commonly used in general cosmetic compositions.

When the cosmetic composition is in the form of a paste, a cream, or a gel, animal oils, vegetable oils, waxes, paraffin, starch, tragacanth, cellulose derivatives, polyethylene glycol, silicone, bentonite, silica, talc, zinc oxide, or the like may be used as carrier components.

When the cosmetic composition is in the form of a powder or a spray, lactose, talc, silica, aluminum hydroxide, calcium silicate, or polyamide powder may be used as carrier components, and particularly in the case of a spray, a propellant such as chlorofluorohydrocarbon, propane/butane, or dimethyl ether may be additionally included, but is not limited thereto.

When the cosmetic composition is in the form of a solution or an emulsion, a solvent, a solubilizer, or an emulsifier may be used as carrier components, and examples thereof include water, ethanol, isopropanol, ethyl carbonate, ethyl acetate, benzyl alcohol, benzyl benzoate, propylene glycol, 1,3-butylene glycol oil, glycerol aliphatic esters, polyethylene glycol, or fatty acid esters of sorbitan.

When the cosmetic composition is in the form of a suspension, liquid diluents such as water, ethanol, or propylene glycol, suspending agents such as ethoxylated isostearyl alcohol, polyoxyethyl sorbitol esters, and polyoxyethylene sorbitan esters, microcrystalline cellulose, aluminum metahydroxide, bentonite, agar, tragacanth, or the like may be used as carrier components.

When the cosmetic composition is in the form of a surfactant-containing cleanser, aliphatic alcohol sulfates, aliphatic alcohol ether sulfates, sulfosuccinic acid monoesters, isethionates, imidazolinium derivatives, methyl taurates, sarcosinates, fatty acid amide ether sulfates, alkylamidobetaines, aliphatic alcohols, fatty acid glycerides, fatty acid diethanolamides, vegetable oils, lanolin derivatives, ethoxylated glycerol fatty acid esters, or the like may be used as carrier components.

When the cosmetic composition is in the form of a hair shampoo, base ingredients for formulating a shampoo, such as a thickening agent, a surfactant, a viscosity modifier, a moisturizer, a pH adjuster, a preservative, and essential oils, may be mixed. As the thickening agent, CDE may be used; as the surfactant, an anionic surfactant, LES, and an amphoteric surfactant, coco betaine, may be used; as the viscosity modifier, polyquaternium may be used; as the moisturizer, glycerin may be used; and as the pH adjuster, citric acid or sodium hydroxide may be used. As the preservative, grapefruit extract or the like may be used, and in addition, essential oils such as cedarwood, peppermint, and rosemary, as well as silk amino acids, pentaol, or vitamin E, may be added.

The ingredients contained in the cosmetic composition may further include, in addition to the peptide of the present invention as an active ingredient and carrier components, ingredients commonly used in cosmetic compositions, for example, conventional auxiliaries such as antioxidants, stabilizers, solubilizers, vitamins, pigments, or fragrances, but are not limited thereto.

According to another aspect of the present invention, there is provided a functional food composition for improving a skin condition, the functional food composition containing, as an active ingredient, a peptide including the amino acid sequence set forth in SEQ ID NO: 1.

The improvement of the skin condition may be improvement in skin aging, promotion of skin regeneration, improvement in skin elasticity, improvement in skin wrinkles, or improvement in a skin barrier.

In the functional food composition of the present invention, activities related to improving a skin condition are the same as those described in the above-described composition for improving a skin condition, and thus a redundant description thereof is omitted.

In the functional food composition of the present invention, the peptide, which is an active ingredient, may be contained in an amount of 10 wt% or less based on the total weight of the composition, specifically in an appropriate amount within a range of 0.000001 wt% to 10 wt%.

The functional food composition of the present invention may contain a food-effective amount of the peptide and a food-acceptable carrier.

The functional food composition of the present invention may contain, as the active ingredient, not only the peptide but also ingredients that are commonly added during food manufacturing, and may contain, for example, proteins, carbohydrates, fats, nutrients, seasonings, and flavoring agents. Examples of the carbohydrates described above include common sugars, such as monosaccharides, for example, glucose and fructose; disaccharides, for example, maltose, sucrose, and oligosaccharides; and polysaccharides, for example, dextrin and cyclodextrin; and sugar alcohols such as xylitol, sorbitol, and erythritol. As flavoring agents, natural flavoring agents, thaumatin, stevia extracts (for example, rebaudioside A, glycyrrhizin, and the like), and synthetic flavoring agents (saccharin, aspartame, and the like) may be used.

The functional food composition of the present invention may contain, in addition to the ingredients described above, various nutritional supplements, vitamins, minerals (electrolytes), flavoring agents such as synthetic flavoring agents and natural flavoring agents, colorants, bulking agents (cheese, chocolate, and the like), pectic acid and salts thereof, alginic acid and salts thereof, organic acids, protective colloid thickeners, pH adjusters, stabilizers, preservatives, glycerin, alcohol, and carbonating agents used in carbonated beverages. In addition, the functional food composition may contain fruit flesh for preparing natural fruit juice, fruit juice beverages, and vegetable beverages. For example, when the functional food composition of the present invention is prepared as a drink, the functional food composition may additionally contain, in addition to the peptide of the present invention as the active ingredient, citric acid, liquid fructose, sugar, glucose, acetic acid, malic acid, fruit juice, Eucommia ulmoides extract, jujube extract, licorice extract, and the like.

There is no particular limitation on the type of the functional food. Examples of the functional food or food include meat, sausages, bread, chocolate, candies, snacks, confectionery, pizza, ramen, other noodles, gums, dairy products including ice creams, various soups, beverages, tea drinks, alcoholic beverages, vitamin complexes, dairy products, fermented milk, and the like, and may include all functional foods or foods in the usual sense.

### Use of Peptide

According to still another aspect of the present invention, there is provided a use of a peptide including the amino acid sequence set forth in SEQ ID NO: 1 for improving a skin condition.

According to still another aspect of the present invention, there is provided a use of a peptide including the amino acid sequence set forth in SEQ ID NO: 1 for the manufacture of cosmetics or functional food for improving a skin condition.

According to still another aspect of the present invention, there is provided a method for improving a skin condition in a patient or subject in need thereof, the method including administering to the patient or subject a peptide including the amino acid sequence set forth in SEQ ID NO: 1 or a composition containing the peptide as an active ingredient.

The term "patient" or "subject" as used herein refers to a human or a non-human animal, for example, a human, a primate, a mammal, or a vertebrate.

In the uses and the method for improving a skin condition described above, the descriptions regarding the peptide of the present invention, activities thereof, and compositions described above may be equally applied, and thus will not be repeated in order to avoid undue complexity in the specification.

### ADVANTAGEOUS EFFECTS

The peptide of the present invention has activity of improving a skin condition. Specifically, the peptide of the present invention increases expression levels of extracellular matrix components and skin barrier enhancing factors, reduces expression of skin cell damage factors that have been increased by ultraviolet radiation again, and restores expression levels of extracellular matrix components and skin barrier enhancing factors that have been reduced by ultraviolet radiation.

However, the effects of the present invention are not limited to the effects mentioned above, and other effects that are not mentioned may be obviously understood by those skilled in the art from the following description.

### BRIEF DESCRIPTION OF THE DRAWINGS

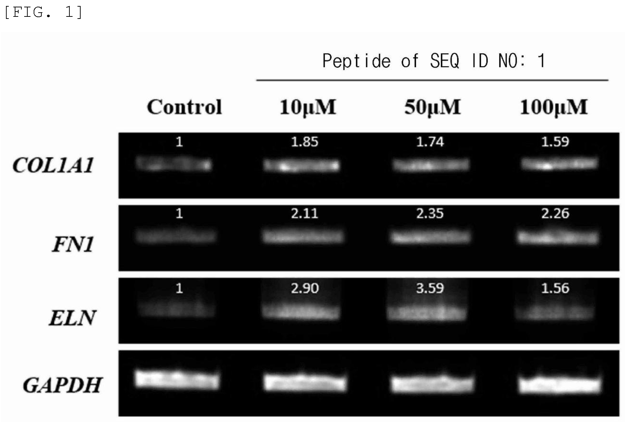

FIG. 1 shows that the peptide of the present invention increases mRNA expression levels of collagen Iα1 (COLA1), fibronectin (FN1), and elastin (ELN) genes in NIH3T3 cells. The numerical values in the drawing represent relative expression levels obtained by calculating the band density value of each gene relative to the band density value of GAPDH as a loading control and setting the control to 1.

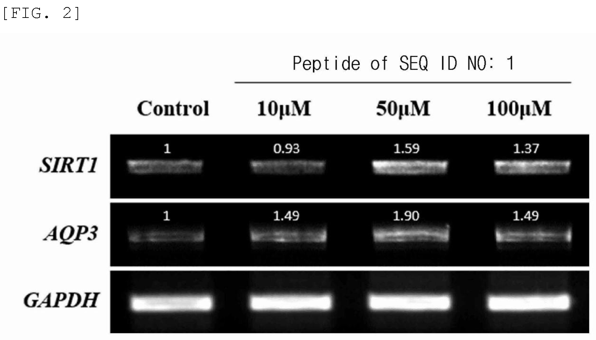

FIG. 2 shows that the peptide of the present invention increases mRNA expression levels of SIRT1 (Sirtuin 1) and AQP3 (Aquaporin 3) genes in HaCaT cells. The numerical values in the drawing represent relative expression levels obtained by calculating the band density value of each gene relative to the band density value of GAPDH as a loading control and setting the control to 1.

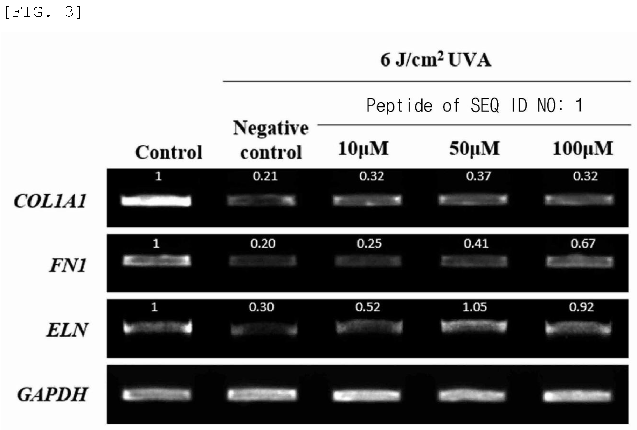

FIGS. 3 shows that the peptide of the present invention again increases mRNA expression levels of collagen Iα1, fibronectin, and elastin genes reduced by UV irradiation in NIH3T3 cells. The numerical values in the drawing represent relative expression levels obtained by calculating the band density value of each gene relative to the band density value of GAPDH as a loading control and setting the control to 1.

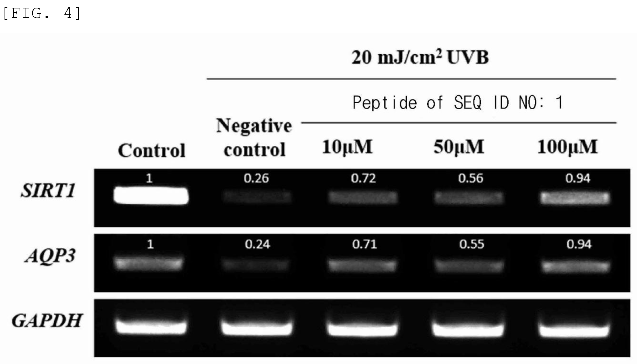

FIG. 4 shows that the peptide of the present invention again increases mRNA levels of SIRT1 and AQP3 genes reduced by UV irradiation in HaCaT cells. The numerical values in the drawing represent relative expression levels obtained by calculating the band density value of each gene relative to the band density value of GAPDH as a loading control and setting the control to 1.

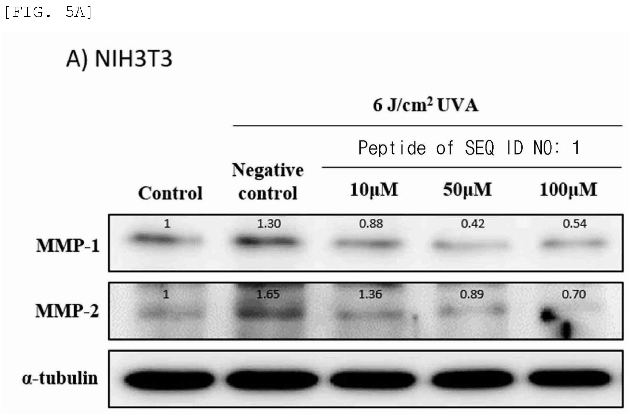

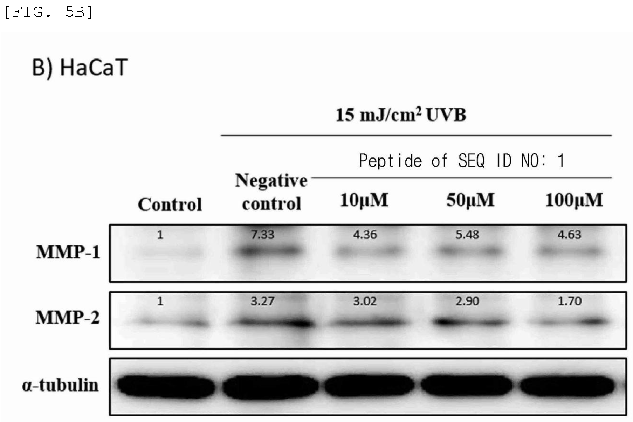

FIGS. 5A and 5B show that the peptide of the present invention again reduces expression of MMP-1 and MMP-2 increased by UV irradiation in NIH3T3 cells and HaCaT cells. The numerical values in the drawing represent relative expression levels obtained by calculating the band density values of MMP-1 and MMP-2 relative to the band density value of α-tubulin as a loading control and setting the control to 1.

### BEST MODE FOR CARRYING OUT THE INVENTION

Hereinafter, the present invention will be described in detail with reference to Examples. However, the following Examples are intended to specifically illustrate the present invention, and the present invention is not limited by the following Examples.

### Preparation Example 1: Preparation of Peptide

Using an automated peptide synthesizer (Milligen 9050, Millipore, USA), a peptide having the amino acid sequence of SEQ ID NO: 1 shown in Table 1 were synthesized, and the synthesized peptide was isolated and purified by C18 reversedphase high-performance liquid chromatography (HPLC) (Waters Associates, USA). As a column, an ACQUITY UPLC BEH300 C18 (2.1 mm X 100 mm, 1.7 µm, Waters Co., USA) was used.

**[Table 1]**

| SEQ ID NO: | Amino acid sequence (N-terminus → C-terminus) |
|---|---|
| 1 | DRKKIQNK |

The efficacy of the peptide of SEQ ID NO: 1 prepared above was evaluated through the following experiments.

### Experimental Example 1: Analysis of Expression of Extracellular Matrix Component Genes

The effect of the peptide prepared in Preparation Example 1 on expression of extracellular matrix (ECM) components was evaluated by measuring mRNA expression levels of the genes of the respective components.

NIH3T3 cells (a mouse fibroblast cell line) were seeded in a 6-well plate at a density of 3 x 10⁵ cells/well and cultured for 24 hours. The cultured cells were washed once with serum-free DMEM. The peptide prepared in Preparation Example 1 was mixed into serum-free DMEM at each concentration to prepare medium solutions containing the peptide at 10 µM, 50 µM, and 100 µM, and the medium solutions were added to the cells. The cells were cultured for 24 hours in a CO₂ incubator at 37°C and washed twice with PBS, and then RNA was isolated using easy blue (iNtRON, Cat. No.: 17061, Korea). The amount of RNA was quantified, 1,000 ng of RNA was added per tube, and then cDNA synthesis was performed using an RT kit (enzynomics, Cat. No.: RT200, Korea). Thereafter, PCR was performed using a PCR kit (enzynomics, Cat. No.: P581T, Korea), with collagen Iα1, fibronectin, or elastin gene as a detection target. The primers used for PCR are shown in Table 2.

As a result of the experiment, it was confirmed that, in NIH3T3 cells, the mRNA expression levels of collagen Iα1 (Collagen, type I, alpha 1), fibronectin, and elastin genes, which are components constituting the extracellular matrix (ECM), were increased by the peptide of SEQ ID NO: 1 (FIG. 1).

### Experimental Example 2: Analysis of Expression of Skin Barrier-Related Genes

The effect of the peptide prepared in Preparation Example 1 on expression of genes related to a skin barrier was evaluated.

HaCaT cells (a human keratinocyte cell line) were seeded in a 6-well plate at a density of 3 x 10⁵ cells/well and cultured for 24 hours. The cultured cells were washed once with serum-free DMEM. The peptide prepared in Preparation Example 1 was mixed into serum-free DMEM at each concentration to prepare medium solutions containing the peptide at 10 µM, 50 µM, and 100 µM, and the medium solutions were added to the cells. The cells were cultured for 24 hours in a CO₂ incubator at 37°C and washed twice with PBS, and then RNA was isolated using easy blue (iNtRON, Cat. No.: 17061, Korea). The amount of RNA was quantified, 1,000 ng of RNA was added per tube, and then cDNA synthesis was performed using an RT kit (enzynomics, Cat. No.: RT200, Korea). Thereafter, PCR was performed using a PCR kit (enzynomics, Cat. No.: P581T, Korea), with SIRT1 (Sirtuin 1) or AQP3 (Aquaporin 3) as a detection target. The primers used for PCR are shown in Table 2.

SIRT1 (Sirtuin 1) and AQP3 (Aquaporin 3) are known as factors that affect maintenance of a skin barrier by regulating aging and moisturization of cells constituting the epidermis. As a result of the experiment, it was confirmed that, in HaCaT cells, the peptide of SEQ ID NO: 1 increased mRNA expression levels of SIRT1 (Sirtuin 1) and AQP3 (Aquaporin 3) genes, which are skin barrier enhancing factors (FIG. 2).

### Experimental Example 3: Restoration of Expression of Extracellular Matrix Genes Reduced by UV Irradiation

Whether the peptide of SEQ ID NO: 1 prepared in Preparation Example 1 restores expression levels of extracellular matrix genes reduced by UV irradiation was evaluated.

NIH3T3 cells (a mouse fibroblast cell line) were seeded in a 6-well plate at a density of 5 x 10⁵ cells/well and cultured for 24 hours. The cultured cells were washed once with serum-free DMEM. The peptide prepared in Preparation Example 1 was mixed into serum-free DMEM at each concentration to prepare medium solutions containing the peptide at 10 µM, 50 µM, and 100 µM, and the medium solutions were added to the cells. The cells to which the peptide was added were cultured for 1 hour in a CO₂ incubator at 37°C. After the cells were cultured, the medium was removed from the cells, and PBS was added. Using a UV irradiator (VILBER LOURMAT, Cat. No.: 3102-BSU, France), the cells were irradiated with UVA at 6 J/cm². After PBS was removed, the above-described peptide-containing medium solutions at the respective concentrations were added again to the cells. The cells to which the peptide was added were cultured for 6 hours in a CO₂ incubator at 37°C. After the cells were washed twice with PBS, RNA was isolated using easy blue (iNtRON, Cat. No.: 17061, Korea). The amount of RNA was quantified, 1,000 ng of RNA was added per tube, and then cDNA synthesis was performed using an RT kit (enzynomics, Cat. No.: RT200, Korea). Subsequently, PCR was performed using a PCR kit (enzynomics, Cat. No.: P581T, Korea), with collagen Iα1, fibronectin, or elastin gene as a detection target. The primers used for PCR are shown in Table 2.

As a result of the experiment, in NIH3T3 cells, mRNA expression levels of collagen Iα1, fibronectin, and elastin genes were reduced by UV irradiation. However, when the cells were treated with the peptide of SEQ ID NO: 1, expression levels of the genes that had been reduced were increased again (FIG. 3).

### Experimental Example 4: Restoration of Expression of Skin Barrier-Related Genes Reduced by UV Irradiation

Whether the peptide of SEQ ID NO: 1 prepared in Preparation Example 1 restores expression levels of skin barrier-related genes reduced by UV irradiation was evaluated.

HaCaT cells (a human keratinocyte cell line) were seeded in a 6-well plate at a density of 5 x 10⁵ cells/well and cultured for 24 hours. The cultured cells were washed once with serum-free DMEM. The peptide prepared in Preparation Example 1 was mixed into serum-free DMEM at each concentration to prepare medium solutions containing the peptide at 10 µM, 50 µM, and 100 µM, and the medium solutions were added to the cells. The cells to which the peptide was added were cultured for 1 hour in a CO₂ incubator at 37°C. After the cells were cultured, the medium was removed from the cells, and PBS was added. Using a UV irradiator (VILBER LOURMAT, Cat. No.: 3102-BSU, France), the cells were irradiated with UVB at 20 mJ/cm². After PBS was removed, the above-described peptide-containing medium solutions at the respective concentrations were added again to the cells. The cells to which the peptide was added were cultured for 4 hours in a CO₂ incubator at 37°C. After the cells were washed twice with PBS, RNA was isolated using easy blue (iNtRON, Cat. No.: 17061, Korea). The amount of RNA was quantified, 1,000 ng of RNA was added per tube, and then cDNA synthesis was performed using an RT kit (enzynomics, Cat. No.: RT200, Korea). Subsequently, PCR was performed using a PCR kit (enzynomics, Cat. No.: P581T, Korea), with SIRT1 (Sirtuin 1) or AQP3 (Aquaporin 3) as a detection target. The primers used for PCR are shown in Table 2.

As a result of the experiment, in HaCaT cells, the mRNA levels of SIRT1 and AQP3 genes were reduced by UV irradiation, and when the cells were treated with the peptide of SEQ ID NO: 1, the mRNA expression levels of these genes that had been reduced were restored (FIG. 4).

### Experimental Example 5: Inhibition of UV-Induced MMP-1 and MMP-2 Expression

The effect of the peptide of SEQ ID NO: 1 prepared in Preparation Example 1 on MMP-1 and MMP-2 expression induced by UV was evaluated.

NIH3T3 cells (a mouse fibroblast cell line) and HaCaT cells (a human keratinocyte cell line) were seeded in a 6-well plate at a density of 5 x 10⁵ cells/well and cultured for 24 hours. The cultured cells were washed once with serum-free DMEM. The peptide prepared in Preparation Example 1 was mixed into serum-free DMEM at each concentration to prepare medium solutions containing the peptide at 10 µM, 50 µM, and 100 µM, and the medium solutions were added to the cells. The cells to which the peptide was added were cultured for 1 hour in a CO₂ incubator at 37°C. After the cells were cultured, the medium was removed from the cells, and PBS was added. Using a UV irradiator (VILBER LOURMAT, Cat. No.: 3102-BSU, France), NIH3T3 cells were irradiated with UVA at 6 J/cm², and HaCaT cells were irradiated with UVB at 15 mJ/cm². After PBS was removed, the above-described peptide-containing medium solutions at the respective concentrations were added again to the cells. The cells to which the peptide was added were cultured for 24 hours in a CO₂ incubator at 37°C. After the cells were washed twice with PBS, a cell lysis buffer was added to lyse the cells. After treatment with a 5X sample buffer, SDS-PAGE was performed using a 10% SDS-PAGE gel. Proteins separated by SDS-PAGE were transferred to a PVDF membrane. Blocking was performed for 1 hour at room temperature using 5% skim milk. An anti-MMP-1 primary antibody (Abcam, Cat. No.: ab137332, UK) and an anti-MMP-2 primary antibody (Abcam, Cat. No.: ab86607, UK) were each diluted at 1:1,000 in 5% skim milk and allowed to react with the membrane for 2 hours. As a loading control, an anti-α-tubulin antibody (Santa Cruz Biotechnology, Cat. No.: sc69969, USA) was allowed to react in the same manner. The membrane was washed three times for 10 minutes each with 0.1% PBS-T (0.1% Tween-20 in PBS). As a secondary antibody against the MMP-1 primary antibody, Goat Anti-Rabbit IgG (H+L) (Jackson ImmunoResearch, Cat. No.: 111-035-003, USA) was diluted at 1:3,000 in 5% skim milk and allowed to react with the membrane for 1 hour. As secondary antibodies against the MMP-2 primary antibody and the α-tubulin primary antibody as a loading control, Goat Anti-Mouse IgG (H+L) (Jackson ImmunoResearch, Cat. No.: 115-035-003, USA) was allowed to react in the same manner. After treatment with an ECL solution (GE Healthcare, Cat. No.: RPN2232, USA), expression levels were analyzed using an ImageQuant 800 instrument (Cytiva, Cat. No.: 29-3994-81, USA).

As a result of the experiment, in NIH3T3 cells, expression of MMP-1 and MMP-2 was increased by UV irradiation, and the peptide of SEQ ID NO: 1 inhibited an increase in MMP-1 and MMP-2 expression induced by UV irradiation (FIG. 5A). In addition, in HaCaT cells, expression of MMP-1 and MMP-2 was increased by UV irradiation, and the peptide of SEQ ID NO: 1 inhibited an increase in MMP-1 and MMP-2 expression induced by UV irradiation (FIG. 5B).

**[Table 2]**

| Primer | Sequence (5' -> 3') | SEQ ID NO: |
|---|---|---|
| Col1a1 Forward | (5') CAC CCT CAA GAG CCT GAG TC (3') | 2 |
| Col1a1 Reverse | (5') AGA CGG CTG AGT AGG GAA CA (3') | 3 |
| Fibronectin Forward | (5') CCA GGA ACC GAG TAC ACC AT (3') | 4 |
| Fibronectin Reverse | (5') ATA CCC AGG TTG GGT GAT GA (3') | 5 |
| Elastin Forward | (5') GCAAGACCTGGCTTTGGACT (3') | 6 |
| Elastin Reverse | (5') GGGAGTTTCTGGTTAGGGCTG (3') | 7 |
| AQP3 Forward | (5') CCT TCT TGG GTG CTG GAA TA (3') | 8 |
| AQP3 Reverse | (5') ACA CGA TAA GGG AGG CTG TG (3') | 9 |
| SIRT1 Forward | (5') TCA GTG GCT GGA ACA GTG AG (3') | 10 |
| SIRT1 Reverse | (5') TCT GGC ATG TCC CAC TAT CA (3') | 11 |
| GAPDH Forward | (5') GGA GCC AAA AGG GTC ATC AT (3') | 12 |
| GAPDH Reverse | (5') GTG ATG GCA TGG ACT GTG GT (3') | 13 |

Although representative embodiments of the present invention have been described above by way of example, the scope of the present invention is not limited to the specific embodiments described herein. Appropriate modifications may be made by those skilled in the art within the scope of the claims of the present invention.

## Claims

1. A peptide comprising the amino acid sequence of SEQ ID NO: 1.

2. A composition for improving a skin condition, the composition comprising the peptide of claim 1 as an active ingredient.

3. The composition of claim 2, wherein the improving of the skin condition is improvement in skin aging, promotion of skin regeneration, improvement in skin elasticity, improvement in skin wrinkles, or improvement in a skin barrier.

4. The composition of claim 2, wherein the composition has a skin condition-improving activity through one or more of the following activities:
(i) increasing expression levels of collagen Iα1, fibronectin, and elastin genes, which are extracellular matrix components;
(ii) increasing expression levels of SIRT1 (Sirtuin 1) and AQP3 (Aquaporin 3) genes, which are skin barrier enhancing factors;
(iii) increasing expression levels of collagen Iα1, fibronectin, and elastin genes, which are extracellular matrix components, the expression levels being reduced by UV irradiation;
(iv) increasing expression levels of SIRT1 and AQP3 genes, which are skin barrier enhancing factors, the expression levels being reduced by ultraviolet irradiation; and
(v) reducing expression levels of MMP-1 and MMP-2 increased by ultraviolet irradiation.

5. The composition of claim 2, wherein the composition is a cosmetic composition.

6. The composition of claim 5, wherein the cosmetic composition is in a form selected from the group consisting of a solution, a suspension, an emulsion, a paste, a gel, a cream, a lotion, a powder, a soap, a surfactant-containing cleanser, an oil, a powder foundation, an emulsion foundation, a wax foundation, and a spray.

7. A functional food composition for improving a skin condition, the functional food composition comprising, as an active ingredient, a peptide comprising the amino acid sequence of SEQ ID NO: 1.

8. The functional food composition of claim 7, wherein the improving of the skin condition is improvement in skin aging, promotion of skin regeneration, improvement in skin elasticity, improvement in skin wrinkles, or improvement in a skin barrier.

9. A method for improving a skin condition in a patient or subject in need thereof, the method comprising administering to the patient or subject a peptide comprising the amino acid sequence of SEQ ID NO: 1 or a composition comprising the peptide as an active ingredient.
